# EUROPEAN PATENT SPECIFICATION

(11) **EP 1 458 331 B1**
(45) Date of publication and mention of the grant of the patent: **06.08.2008**
(21) Application number: 02781619.8
(22) Date of filing: 11.12.2002
(51) Int. Cl.: A61K 8/02, A61K 8/37, A61Q 1/08, A61Q 1/10, A61Q 1/12

(54) **PROCESS FOR THE PREPARATION OF COSMETICS**
VERFAHREN ZUR HERSTELLUNG VON KOSMETISCHEN ZUSAMMENSETZUNGEN
PROCEDE DE PREPARATION DE PRODUITS COSMETIQUES

(30) Priority: 28.12.2001 IT MI20012841
(43) Date of publication of application: 22.09.2004
(73) Proprietor: GAMMA CROMA S.P.A., 20121 Milan (IT)
(72) Inventor: ANCOROTTI, Renato, I-26013 Crema (IT)
(74) Representative: Trupiano, Federica
(86) International application number: PCT/IB2002/005257
(87) International publication number: WO 2003/055453

(56) References cited:
- EP-A- 0 021 135
- EP-A- 0 734 733
- EP-A- 0 756 865
- GB-A- 2 048 296
- US-A- 4 994 264
- DATABASE CHEMICAL ABSTRACTS [Online] retrieved from STN Database accession no. 112:223139 XP002245077 & JP 01 211512 A (POLA CHEM. IND., INC.) 24 August 1989 (1989-08-24)
- DATABASE CHEMICAL ABSTRACTS [Online] retrieved from STN Database accession no. 119: 278379 XP002245078 & JP 05 229917 A (TONBO PENCIL) 7 September 1993 (1993-09-07)

## Description

### Technical field

This invention concerns a new process for the preparation of make-up cosmetics such as eye shadow, face powder and blusher.

In particular, the invention concerns a process for the preparation of a cosmetic with a solid consistency and an excellent powdery marking effect, said process comprising extruding and drying a paste-type mixture and subsequent processing the solid product obtained.

### Background of the invention

Face and body make-up cosmetics such as blusher and eye shadow are known. These products are normally produced in the form of pressed or non-pressed powders.

Both these processes result in products that offer both advantages and disadvantages, for example:
- pressed powders have the advantage that they are easy to apply but on the other hand after being used for some time they tend to crumble, dirtying the container and the objects with which they come into contact;
- non-pressed powders require a certain amount of skill for their application as they are designed for a more professional use but the containers wherein they are packaged are provided with fastenings that prevent the product leaking out.

US 4 994 264 discloses the preparation of a paste to be extruded comprising a gum containing aqueous composition, especially xanthan gum, said paste being made by mixing all the components one by one.

EP 21 135 A2 discloses a solid stick cosmetic composition to be used as a cream, which comprises a mixture of fats, emulsifiers, water soluble binders and water, such a mixture being extruded and dried.

Chemical Abstracts 119:278379 (JP05229917) discloses the manufacture of an eyebrow pencil wherein an oil-in-water emulsion is prepared and mixed up with a solution containing hydroxyethylcellulose in water and dyes, then extruded to give a lead.

### Summary of the Invention

It has now surprisingly been found that via a multi-phase process, comprising mixing, extruding and drying, make-up cosmetics can be obtained free from the problems of the conventional products and with a solid consistency so that they can be produced in different formats; the process also results in products that are easy to apply and at the same time have a good powdery marking effect.

Thus, according to one of its aspects, the invention concerns a process for the preparation of a cosmetic that comprises extruding a paste obtained by mixing at least fats for cosmetic use, colouring powders and at least one solvent for cosmetic use, and subsequently drying the extruded product.

### Detailed description of the invention

According to one embodiment, the invention concerns a process for the preparation of extruded powders for cosmetics that comprises extruding of a paste obtained by mixing an emulsion of fats for cosmetic use with colouring powders and drying of the extruded product.

In further detail the invention concerns a process for the preparation of extruded powders for cosmetics that comprises:
a) preparing the two phases, herein called "colouring powders" and "fatty emulsion";
b) mixing said phases;
c) extruding the paste obtained;
d) drying the extruded product;
e) sizing the dried product if required.

The "fatty emulsion" according to this invention can be obtained by treating fats for cosmetic use with at least one solvent, for example water or any solvent suitable for cosmetic use, including their mixtures; the essential feature of the solvent used is that it is possible to eliminate it by drying after extrusion at temperatures that do not alter the end product, advantageously at temperatures not exceeding 50°C.

The solvent can be neutral or coloured, in the latter case either due to its specific properties or by the addition of colouring substances. Water, being readily available and inexpensive, is the preferred solvent for this invention.

According to this invention, the expression "fats for cosmetic use" indicates any fatty material suitable for the preparation of cosmetics such as the esters of fatty acids, triglycerides, waxes, fruit and seed oil derivatives and extracts etc.

Fats for cosmetic use useful according to the invention are, for example, sorbitan stearate, isopropyl stearate, caprylic/capric triglycerides, dipentacrythrityl hexahydroxystearate/ stearate rosinate (sold under the trademark Cosmol 168AR), magnesium myristate and olive oil.

According to this invention, the expression "colouring powders" indicates any powder, or mixture of powders, containing colouring pigments suitable for cosmetic use.

Suitable colouring powders are, for example, those obtained by mixing synthetic and/or natural pigments, matte or pearly, with inert powders as diluents such as mica or talc, in varying quantities according to the powdery effect and colouring power required.

The pearly and colouring substances that can be used include the following, for example:
TiO2 (Cl 77891) + mica (Cl 77019)
Bismuth oxychloride Cl 77163
Mica Cl 77019
Copper and bronze powder Cl 7740
Iron oxide Cl 77491-2-9
Ultramarine blue Cl 77007
Manganese violet Cl 77742
Chromium hydrate oxide Cl 77289
Anhydrous chromium oxide Cl 77288
Ferric ferrocyanide Cl 77510
Titanium dioxide Cl 77891
D&C red no. 7 Ca lake Cl 15850:1
D&C red no. 19 Al lake Cl 45170:3
D&C red no. 6 Ba lake Cl 15850:2
D&C red no. 3 Al lake Cl 45430:1
D&C red no. 9 Ba lake Cl 15585:1
D&C red no. 21 Al lake Cl 45380:3
D&C yellow no. 5 Al lake Cl 19140:1
D&C red no. 30 Al lake Cl 73360
D&C yellow no. 10 Al lake Cl 47005:1
D&C red no. 27 Al lake Cl 45410:2
D&C yellow no. 5 Al lake Cl 19140:1
D&C orange 5 Cl 45370:1
FD&C yellow no. 6 Al lake Cl 15985:1
FD&C blue no. 1 Al lake Cl 42090:2
D&C red 36 Cl 12085
Carmine Cl 75470

It is evident that the paste to be extruded can be obtained either by mixing each individual component, one after the other, in a mixer for example, or by first preparing the two "fatty emulsion" and "colouring powders" phases and then mixing them.

The quantity of individual components can vary within a wide range according to the type of product required.

The "fatty emulsion" and "colouring powders" phases are preferably mixed in equal quantities, i.e. 50-50% by weight.

Extruding process of this invention can be performed by passing the paste (neutral or coloured) obtained in phase (b) through a normal extruder (or drawing machine) to obtain a semi-solid product, still damp, of the required shape.

The extruders useful in the process of the invention are, for example, piston or screw extruders. The product comes out of the extruder in different shapes according to the dies used (for example cylinder, parallelepiped etc.) and is cut to the required length.

The drying phase of the process of the invention can be performed according to the conventional techniques, for example:
- in an oven
- under vacuum, or
- with fluidised bed
provided that said process creates conditions that ensure the evaporation of almost all the solvent used for preparation of the paste to be extruded, whatever it is. Obviously lower temperatures require longer drying times whereas at higher temperatures drying times will be shorter. The drying (or baking) will be preferably performed at low temperatures in order to permit slow elimination of the solvent from the paste and to obtain a well-pressed and uniform end product, without altering the end product.

Advantageously, the drying is performed in an oven at temperatures of 35-55°C, around 40°C for example, until almost complete evaporation of the solvent, for example until the residual humidity is below or equal to 5%.

According to a preferred embodiment, the invention concerns a process for the preparation of extruded powder for cosmetics comprising:
- preparation of a paste in a mixer by mixing an emulsion of fats for cosmetic use with colouring powders;
- extrusion of the paste formed as above by means of an extruder or drawing machine according to various shapes and sizes;
- drying of the extruded products in an oven at a temperature of between 35 and 55°C until a residual humidity lower than or equal to 5% is obtained;
- if required or necessary, further processing of the dried product to give it the appropriate shape and size.

The machines and processing techniques individually used in the process of the invention are known to the skilled in the art.

Other components, inert or non-inert, can be added to the colouring powders and fatty emulsion phases of the invention or directly to the paste to be extruded. As an example, additives can be added, as solid and liquid preserving agents, such as sodium benzoate, thickeners such as starch and its derivatives, diluents, gelling agents and adhesives, in order to make the paste obtained by mixture of the two phases suitable for extrusion, various inert powders, fragrances if required, etc. Said additives and their properties and uses in the field of cosmetics are well known to the skilled in the art.

Examples of additives are given in the experimental part.

The colouring powders are generally prepared by mixing the required pigments with inert components such as talc, silica and mica. According to a preferred embodiment, parabenes and sodium dehydroacetate are added to the mixture to preserve the entire product during all the processing phases.

These components are advantageously ground for a few minutes to obtain a uniform powder.

The emulsion and the colouring powders prepared as above are then processed in a mixer and subsequently transferred to an extruder, where the dies give them the required shapes. At the extruder outlet, the product is placed preferably on sheets of inert material, wherein "inert" means that it does not react with the product, for example PVC, steel or similar.

Extrusion is followed by drying, preferably in an oven, normally at a temperature of around 40-50°C for a time that depends on the evaporation speed, generally a few days.

Once drying has been completed, the product can be visually checked to identify any defects and hardness can be checked by means of a dynamometer.

The cosmetics prepared in this way can be further processed if necessary in order to reduce their dimensions or modify their shape, for example. These further operations can be performed manually or by means of appropriate machine tools.

The process of the invention therefore permits the production of cosmetics for decorative or curative make-up with the following characteristics:
- optimal consistency permitting direct application
- excellent marking effect and, at the same time,
- a creamy finish even though the products are powder-based.

The products obtained by means of the process of the invention therefore represent a valid alternative to the conventional products such as in pressed or non-pressed powders and creamy type products.

The following examples are provided for the purpose of illustrating the invention without limiting it in any way.

### EXAMPLE 1

### GENERAL OPERATING METHOD

Appropriately clean and sanitise all the machinery required for the work process.

### Preparation of the fatty emulsion

Weigh the water in an emulsifier; add the selected preservative and mix until fully dispersed. Add any thickeners and the mixture of liquid preservatives and disperse by means of a homogeniser. Weigh and dissolve the fatty mass (stearates, triglycerides) at approximately 60°C, add it to the previously prepared mixture and homogenise. Add the gelling agent and process for a few minutes. Check the viscosity and pour into clean drums.

### Preparation of the colouring powders

Weigh the inert components, binders and any preservatives (talc, parabenes etc.) and grind for a few minutes. Weigh and add the raw materials making up the required colour and grind until obtaining a uniform mixture.

### Preparation of the paste to be extruded and drying

Mix the fatty emulsion and colouring powders for approximately 10 minutes in a mixer. Empty into clean bags and extrude in the drawing machine. Bake the extruded product for 3 days at approximately 50°C.

### EXAMPLE 2

| Qualitative-quantitative composition of a typical paste before extrusion | |
|---|---|
| Water | 39.40% |
| Fats for cosmetic use (mixture of isopropyl stearate, di-C12-13 alkyl malate, Cosmol 168AR) | 6.00% |
| Pigments in admixture with mica | 38.4% |
| Xanthan gum | 0.05% |
| Preservatives (mixture of sorbic acid and parabenes) | 1.1 % |
| Inert components (mixture of zea mays, nylon powder, talc) | 14.55% |
| Fragrance | 0.5% |

## Claims

1. Process for the preparation of extruded powders for cosmetics that comprises:
(a) preparing two phases:
(a') a "fatty emulsion" phase, made of a solvent for cosmetic use and fats for cosmetic use chosen from fatty acids, triglycerides, waxes, and fruit and seed oil derivatives and extracts;
(a") a "colouring powders" phase, made of a powder or a mixture of powders, containing colouring pigments;
(b) mixing said two phases to obtain a paste;
(c) extruding said paste to obtain an extruded product;
(d) drying said extruded product to obtain a dried product;
(e) further sizing said dried product, if required.

2. Process according to claim 1, wherein said fats are chosen from sorbitan stearate, isopropyl stearate, caprylic/capric triglycerides, dipentaerythrityl hexahydroxystearate/ stearate rosinate (Cosmol 168AR), magnesium myristate and olive oil.

3. Process according to claims 1 or 2, wherein said solvent for cosmetic use is water.

4. Process according to claims 1 to 3, wherein said fatty emulsion phase further comprises one or more solid or liquid additives chosen from preserving agents, thickeners, adhesives, diluents and gelling agents.

5. Process according to claims 1 to 4, wherein said colouring powders phase comprises synthetic and/or natural pigments, matte or pearly, with inert powders as diluents.

6. Process according to claims 1 to 5, wherein said fatty emulsion and colouring powders phases are mixed in a proportion of approximately 50-50% by weight.

7. Process according to claims 1 to 6, wherein extruding is performed with an extruder or a drawing machine according to various shapes and sizes.

8. Process according to claims 1 to 7, wherein drying is performed in an oven, under vacuum or with fluidised bed.

9. Process according to claim 8 wherein drying is performed in an oven at a temperature between 35 and 55°C, until obtaining a residual humidity below or equal to 5%.

10. Extruded powders for make-up obtained via the process of any one of the previous claims.

11. Extruded powders of claims 10 which is an eye shadow or blusher.

12. Use of the extruded powders obtained via the process of any one of the claims from 1 to 9 for make-up.

## Patentansprüche

1. Verfahren zur Herstellung von extrudierten Pulvern für Kosmetika, welches umfasst:
(a) Zubereitung zweier Phasen:
(a') eine "Fettemulsion"-Phase, hergestellt aus einem Lösungsmittel zur kosmetischen Verwendung sowie Fetten zur kosmetischen Verwendung, ausgewählt unter Fettsäuren, Triglyceriden, Wachsen sowie Derivaten und Extrakten von Frucht- und Samenölen;
(a") eine "Färbepulver"-Phase, hergestellt aus einem Pulver oder einer Mischung von Pulvern, enthaltend Farbpigmente;
(b) Mischen der zwei Phasen, um eine Paste zu erhalten;
(c) Extrudieren dieser Paste, um ein extrudiertes Produkt zu erhalten;
(d) Trocknen des extrudierten Produkts, um ein getrocknetes Produkt zu erhalten;
(e) ferner Klassieren des getrockneten Produkts, falls erforderlich.

2. Verfahren nach Anspruch 1, wobei die Fette ausgewählt werden unter Sorbitanstearat, Isopropylstearat, Capryl-/Caprintriglyceriden, Dipentaerythritylhexa-(hydroxystearat / stearat / resinat) (Cosmol 168AR), Magnesiummyristat und Olivenöl.

3. Verfahren nach Anspruch 1 oder 2, wobei das Lösungsmittel zur kosmetischen Verwendung Wasser ist.

4. Verfahren nach Anspruch 1 bis 3, wobei die Fettemulsion-Phase ferner ein oder mehrere feste oder flüssige Additive umfasst, ausgewählt unter Konservierungsmitteln, Verdickungsmitteln, Haftmitteln, Verdünnungsmitteln und Geliermitteln.

5. Verfahren nach Anspruch 1 bis 4, wobei die Färbepulver-Phase synthetische und/oder natürliche Pigmente, matt oder perlglänzend, umfasst, mit inerten Pulvern als Verdünnungsmitteln.

6. Verfahren nach Anspruch 1 bis 5, wobei die Fettemulsion- und die Färbepulver-Phasen in einem Verhältnis von etwa 50:50 Gewichtsprozent gemischt werden.

7. Verfahren nach Anspruch 1 bis 6, wobei das Extrudieren durchgeführt wird mit einem Extruder oder einer Ziehmaschine, entsprechend verschiedener Formen und Größen.

8. Verfahren nach Anspruch 1 bis 7, wobei das Trocknen durchgeführt wird in einem Ofen, unter Vakuum oder mit Wirbelbett.

9. Verfahren nach Anspruch 8, wobei das Trocknen in einem Ofen bei einer Temperatur zwischen 35 und 55°C durchgeführt wird, bis eine Restfeuchte von kleiner oder gleich 5 % erhalten wird.

10. Extrudierte Pulver für Schminke, erhalten durch das Verfahren nach einem der vorhergehenden Ansprüche.

11. Extrudierte Pulver nach Anspruch 10, welches ein Lidschatten oder Rouge ist.

12. Verwendung der extrudierten Pulver, erhalten durch das Verfahren nach einem der vorhergehenden Ansprüche 1 bis 9, für Schminke.

## Revendications

1. Procédé de préparation de poudres extrudées pour des produits cosmétiques qui comprend :
(a) la préparation de deux phases :
(a') un phase en « émulsion grasse » constituée d'un solvant pour une utilisation cosmétique et de graisses pour une utilisation cométique choisies parmi les acides gras, les triglycérides, les cires et les dérivés et les extraits d'huile de fruit et de coton ;
(a") un phase de « poudre colorante » constituée d'une poudre ou d'un mélange de poudres, contenant des pigments colorants ;
(b) le mélange desdites deux phases pour obtenir une pâte ;
(c) l'extrusion de ladite pâte pour obtenir un produit extrudé ;
(d) le séchage dudit produit extrudé pour obtenir un produit sec ;
(e) en outre le calibrage dudit produit sec, si nécessaire.

2. Procédé selon la revendication 1, dans lequel lesdites graisses sont choisies parmi le stéarate de sorbitane, le stéarate d'isopropyle, les triglycérides capryliques/capriques, l'hexahydroxystéarate de dipentaérythrityle/stéarate rosinate (Cosmol 168AR), le myristate de magnésium et l'huile d'olive.

3. Procédé selon la revendication 1 ou 2, dans lequel ledit solvant pour une utilisation cosmétique est l'eau.

4. Procédé selon les revendications 1 à 3, dans lequel ladite phase en émulsion grasse comprend en outre un ou plusieurs additifs solides ou liquides choisis parmi les agents de conservation, les épaississants, les adhésifs, les diluants et les agents gélifiants.

5. Procédé selon les revendications 1 à 4, dans lequel ladite phase de poudre colorante comprend les pigments synthétiques et/ou naturels, mats ou nacrés, avec des poudres inertes comme diluants.

6. Procédé selon les revendications 1 à 5, dans lequel lesdites phases en émulsion grasse et de poudre colorante sont mélangées en une proportion d'environ 50-50 % en poids.

7. Procédé selon les revendications 1 à 6, dans lequel l'extrusion est réalisée avec une extrudeuse ou une machine à façonner selon diverses formes et tailles.

8. Procédé selon les revendications 1 à 7, dans lequel le séchage est réalisé dans un four, sous vide ou avec un lit fluidisé.

9. Procédé selon la revendication 8, dans lequel le séchage est réalisé dans un four à une température de 35 à 55 °C, jusqu'à l'obtention d'une humidité résiduelle inférieure ou égale à 5 %.

10. Poudres extrudées pour du maquillage obtenues par le procédé selon l'une quelconque des revendications précédentes.

11. Poudres extrudées selon la revendication 10, qui sont un fard ou une ombre à paupière.

12. Utilisation de poudres extrudées obtenues par le procédé selon l'une quelconque des revendications 1 à 9, pour du maquillage.
